# EUROPEAN PATENT APPLICATION

(11) **EP 1 806 355 A1**
(43) Date of publication of application: **11.07.2007**
(21) Application number: 05788301.9
(22) Date of filing: 30.09.2005
(51) Int. Cl.: C07K 5/08

(54) **PROCESS FOR PRODUCTION OF (1,3-DISUBSTITUTED INDOLYL)- UREA DERIVATIVES, INTERMEDIATES THEREFOR, AND PROCESS FOR PRODUCTION OF THE INTERMEDIATES**

(30) Priority: 30.09.2004 US 614299 P
(71) Applicant: Kowa Company. Ltd., Nagoya-shi, Aichi-ken 460-8625 (JP)
(72) Inventor: KABEYA, Mototsugu, 1890022 (JP); YASUOKA, Kyoko, 2070012 (JP)
(74) Representative: Albrecht, Thomas
(86) International application number: PCT/JP2005/018146
(87) International publication number: WO 2006/035937

(57) **Abstract**

A process for the production of (1,3-disubstituted indolyl) urea derivatives represented by the general formula (1), **characterized by** reacting a 1-substituted-aminoindole derivative with a phenyl halocarbonate to form a carbamate derivative, subjecting the carbamate derivative to Mannich reaction with a secondary amine and formaldehyde to form a 3-aminomethylindole derivative represented by the general formula (6), and then reacting the derivative (6) with a peptide derivative; indole derivatives represented by the general formula (6) which are intermediates for the above process; and a process for the production of the indole derivatives.

## Description

### Technical Field

The present invention relates to a method for producing a (1,3-disubstituted indolyl)urea derivative which is a PAR (protease-activated receptor) - 2 antagonist useful for preventing development and progress of, improving the pathological conditions of, and treating PAR-2 associated diseases including respiratory dysfunction such as asthma, allergic disorders such as allergic rhinitis, cardiovascular events such as myocardial infarction, neurological diseases such as neuralgia, inflammatory diseases such as atopic dermatitis and chronic arthritis, and cancers. The invention further relates to an intermediate thereof as well as a method for producing the same.

### Background Art

PAR-2 is known to be widely distributed in endothelial tissue, is shown to be expressed at particularly high levels in digestive organs, respiratory organs, blood vessels, skin, kidney and the like, and is suggested to be likely to participate widely in inflammatory diseases because it is activated by trypsin, mast cell-derived tryptase or the like in a living body (Pharmacological Rev, 53, 245-282, (2001)). Actually, pharmacological and genetic analysis in recent years using PAR-2 activating peptides or PAR-2 knockout mice have revealed that the stimulation of PAR-2 exhibits an inflammatory action on many organs (Br J Pharmacol, 125, 419-422 (1998)); the expression of PAR-2 is induced by inflammatory stimulation (J Biol Chem., 271(25):14910-14915, (1996)); PAR-2 is expressed at high levels in inflammatory tissues, atherosclerotic plaques, cancer cells, or the like (J Clin Invest., 111(1):35-41, (2003), Int J Oncol., 23(1):61-66 (2003), or the like) ; in the PAR-2 knockout mice, a development of inflammations is suppressed in a contact dermatitis model or in an experimental arthritis model (International Publication WO03/049723) and topical infiltration of inflammatory cells causing asthma is suppressed (J Immunol., 165(11):6509-6510 (2000)) or the like, and the action of PAR-2 on inflammations and cancers attracts attention.

Accordingly, it is estimated that the prevention of development and progress or amelioration of clinical state for inflammatory diseases (asthma, allergic rhinitis, atopic dermatitis, chronic arthritis and the like) and cancers is made feasible by inhibiting the activation of PAR-2, and the development of PAR-2 activation inhibitors, particularly PAR-2 antagonists, as novel anti-inflammatory agents and anticancer drugs is expected.

Until now, a report on PAR-2-selective highly active agonists (International Publication WO03/104268) is recognized, but a report on compounds evidently having a PAR-2 antagonist activity is hardly recognized. Up to now, a compound inhibiting an intracellular signal transduction by stimulation with a PAR-2 agonist has been reported (Japanese Patent Application Laid-open No. 2003-286171), however whether the action of the compound is a direct inhibitory action on PAR-2, is not revealed. A series of antagonists described as being derived from PAR-2 agonist structures have been reported (International Publication WO2004/002418), however their inhibitory activity cannot be said to be satisfactory because the mechanism of PAR-2 inhibition is not revealed and further because the concentration thereof for inhibiting PAR-2 stimulation is shown to be in the order of mM.

In addition to those described above, peptides derived from PAR-1 or PAR-2 activating peptides reported by Al-Ani et al. are reported to suppress the activation of PAR-2 by stimulation with trypsin, but do not exhibit an inhibitory effect on PAR-2 activating peptides, and are suggested to inhibit the binding or interaction between trypsin and PAR-2 . As another unique method of inhibiting PAR-2 activation, there is an approach to specifically inhibit the signal transduction by preventing the receptor from binding to G protein by using a compound (Pepducin) having palmitic acid added to a peptide mimicking an intracellular domain structure of PAR-2 receptor (Nat Med. 2002 Oct; 8(10):1161-5), but a use of this approach as pharmacotherapy still has a problem with respect to suitable delivery of the compound to a target site, specificity of receptor signal or the like. In addition, a low-molecular PAR-2 antagonist acting competitively on a ligand site of the receptor by inhibiting the activation of the PAR-2 accurately is not reported.

### Disclosure of Invention

In view of such circumstances as above, the present inventors conducted an extensive study and consequently found that a compound represented by the general formula (A) or a salt or a solvate thereof, which strongly suppresses the signal transduction of human keratinocyte induced by PAR-2 agonist, has an inhibitory effect on PAR-2.

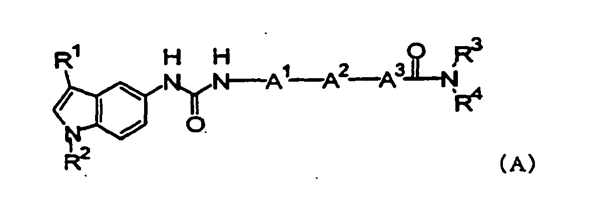

, wherein R¹ is a hydrogen atom, a halogen atom or a group represented by the following formula B:

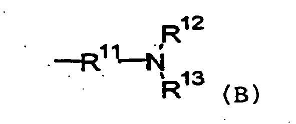

, wherein R¹¹ represents a C₁-C₆ straight-chain or branched alkylene group, and R¹² and R¹³ are taken together with an adjacent nitrogen atom to form a 5 to 7 membered ring, 1 to 2 carbon atom(s) in the ring may be substituted with a nitrogen atom or an oxygen atom, and the ring may be substituted with a C₁-C₆ straight-chain or branched alkyl group;
R² represents a C₁-C₆ straight-chain or branched alkyl group, a C₃-C₆ cycloalkyl group, a C₁-C₆ straight-chain or branched alkyl group substituted with a C₃-C₆ cycloalkyl group, or a C₇-C₁₂ aralkyl group which may be substituted with 1 to 3 subtituents selected from a halogen atom and a C₁-C₆ straight-chain or branched alkyl group;
each of R³ and R⁴ independently represents a hydrogen atom, or a C₇-C₁₂ aralkyl group which may be substituted with 1 to 3 halogen atoms or a C₁-C₆ straight-chain or branched alkyl group; and
each A¹-A²-A³ independently represents a tripeptide residue consisting of α-amino acids selected from the group consisting of glycine, alanine, cyclohexylalanine, α,γ-diaminobutyric acid, lysine, arginine, phenylalanine, valine, or naphthylalanine.

A compound represented by the following formula (A1) among the compounds and the related compounds represented by the general formula (A), can be prepared by reacting a compound (C) with a compound (D) as shown the below reaction scheme.

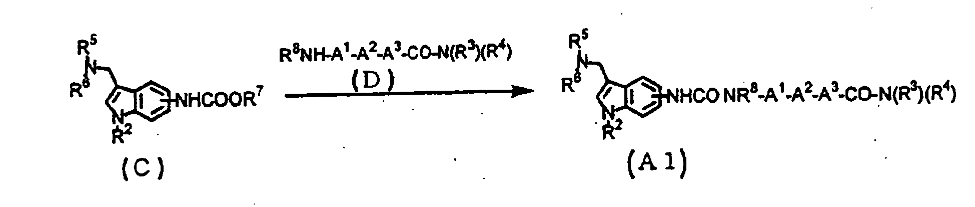

, wherein each of R⁵ and R⁶ independently represents a C₁-C₆ straight-chain or branched alkyl group, or R⁵ and R⁶ are taken together with an adjacent nitrogen atom to form a 5 to 7 membered ring, 1 or 2 carbon atom(s) in the ring may be substituted with a nitrogen or an oxygen atom, and the ring may be substituted with a C₁-C₆ straight-chain or branched alkyl group;
R⁷ is a phenyl group substituted with substituents selected from a C₁-C₆ straight-chain or branched alkyl group, a C₁-C₆ straight-chain or branched alkyloxy group or a halogen atom;
R⁸ is a hydrogen atom, or a C₁-C₆ straight-chain or branched alkyl group;
R², R³, R⁴ and A¹-A²-A³ are the same as defined above.
The reactions of the above scheme can be conducted according to the known method of producing indazole- and indole-6-substiteted derivatives having a PAR-1 antagonist activity (J. Med. Chem., 2001, 44, 1021-1024) as indicated in the following reaction scheme. 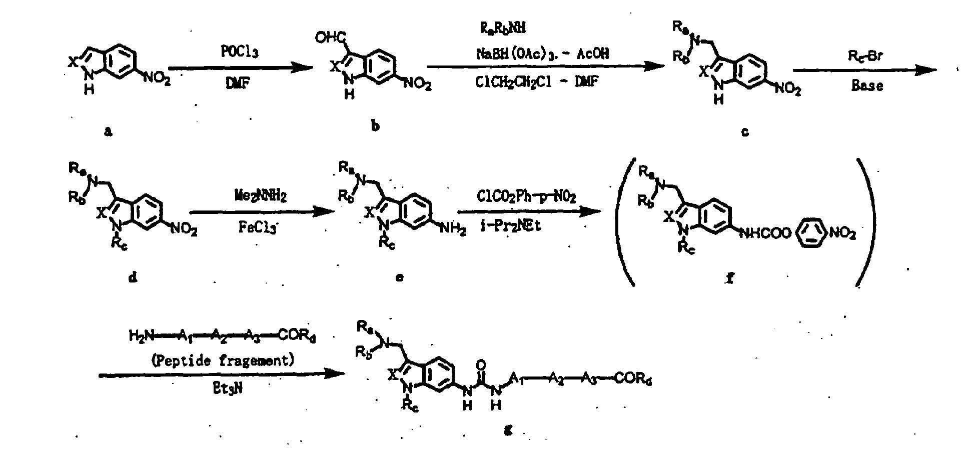 X=N(indazole), CH(indole)

However, as described in the above reference, the total yield in the production process of indazole derivatives is as low as about 6%. Particularly, the production process of compound (g) from (e) cannot be considered as practical due to the low reaction yield and the defects in reproducibility.
The mere application of the present method to a production of the compound (A1) of the present invention results in vein being failed to obtain the desired compound. Since there is no method excellent for producing the compound (A1), the development of a new method is desired.

The present invention is intended to provide a method for producing a (1,3-disubstituted indolyl)urea derivative; as well as to provide an intermediate and the method for producing the same.
In consideration of the above circumstances, the present inventors conducted a diligent study and have succeeded to obtain the compound (1) of interest in high yield and high purity according to the method as shown in the reaction scheme below. The method comprises the following steps: (a) reacting a nitro-indole compound (9) as a starting material with alkyl halide or aralkyl halide represented by R₁X (X is a halogen atom) to form a compound (10); (b) reducing the compound (10) into amino-indole compound (2); (c) reacting the compound (2) with a phenyl halocarbonate derivative represented by R₄OCOX (3) (X is a halogen atom) to form chemically stable phenyl carbamate derivative (4); (d) subj ecting the compound (4) to Mannich reaction with a secondary amine represented by (R₂) (R₃)NH (5) and formalin to form a amino-methyl compound (6); (e) further reacting the compound (6) with a peptide derivative represented by the formula (7).

Alternatively, the method for producing the compound (g) according to the above conventional manner is as follows: a 3-aminomethyl-6-aminoindole derivative (e) is made from the starting material (a); the derivative (e) is reacted with p-nitrophenyl chlorocarbonate under the anhydrous condition to form a carbamate derivative (f) in situ; and, the resulting derivative (f) is reacted with a peptide derivative without isolation to form the compound (g).

However, during the reaction of the compound (e) and p-nitrophenyl chlorocarbonate, the amino-methyl group on 3-position of the compound(e) may induce the side reaction, and the generated carbamate derivative (f) which is quite unstable and cannot be isolated may proceed to a side reaction such as the hydrolysis of the carbamate derivative, and the following homo-coupling reaction of indole derivatives may occur during the consequential reaction of the carbamate derivative with a peptide derivative. The present inventors considered the low yield of the compound (g) is resulted from those side reactions, and thus conducted the investigation over the reaction process and the selection of the intermediates to overcome the above problems for the development of the method of producing the compound (A1).

The present inventors have been succeeded in high-yield and highly selective induction of the aminomethyl group onto the indole ring: firstly the amino-indole derivative (2) is reacted with phenyl halocarbonate derivative (3) to form phenyl carbamate derivative (4), then the Mannich reaction is conducted on 3-position of the indole ring of the derivative. As for the selection of the intermediate, in stead of the unstable *p*-nitrophenyl carbamate derivative,the present inventors have been successfully chosen a phenyl carbamate derivative (4) wherein the phenyl group is not substituted with nitro group. Owing to the proper selection of the stable intermediate with stability and controllable reactivity that can be isolated and purified, the following reaction with the peptide derivative (7) can be carried out by simple procedure in high yield. Consequently, the present inventors have completed the present invention.

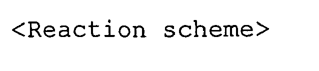

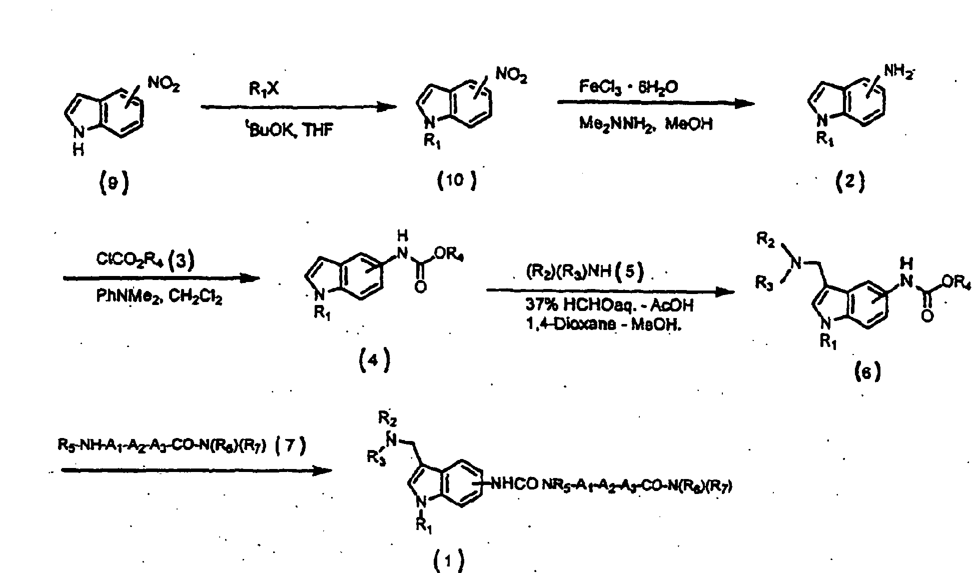

wherein R₁ is a C₁-C₆ straight-chain or branched alkyl group, a C₃-C₆ cycloalkyl group, a C₁-C₆ straight-chain or branched alkyl group, substituted with a C₃-C₆ cycloalkyl group, or a C₇-C₁₂ aralkyl group which may be substituted with ,1 to 3 substituent (s) selected from a halogen atom and a C₁-C₆ straight-chain or branched alkyl group;
each of R₂ and R₃ independently is selected from a C₁-C₆ straight-chain or branched alkyl group, or R₂ and R₃ are taken together with an adjacent nitrogen atom to form 5 to 7 membered ring, 1 to 2 carbon atom(s) in the ring may be substituted with a nitrogen or an oxygen atom, and the ring may be substituted with a C₁-C₆ straight-chain or branched alkyl group;
R₄ is a phenyl group which may be substituted with a C₁-C₆ straight-chain or branched alkyl group, a C₁-C₆ straight-chain or branched alkyloxy group or a halogen atom;
each of R₆ and R₇ independently is a hydrogen atom or a C₇-C₂₁ aralkyl group which may be substituted with 1 to 3 groups selected from a halogen atom, or a C₁-C₆ straight-chain or branched alkyl group;
each A₁-A₂-A₃ independently represents a tripeptide residue consisting of α-amino acid, selected from glycine, alanine, cyclohexylalanine, α,γ-diaminobutyric acid, lysine, arginine, phenylalanine, valine or naphtylalanine;
R₅ is a hydrogen atom, or a C₁-C₆ straight-chain or branched alkyl group;
NO₂ group, NH₂ group, NHCOOR₄ group or the ureylen group is attached to 4,5,6 or 7 position of the indole ring.

Accordingly, the present invention provides a method of producing a (1,3-disubstituted indolyl)urea derivatives (1) and provides the intermediate (6) as well as the method for producing the same, comprising following reaction steps: (a) reacting 1-substituted -aminoindole derivative represented by the general formula (2) with a phenyl halocarbonate compound represented by the general formula (3) to form a carbamate derivative represented by the general formula (4); (b) subjecting the derivative to Mannich reaction with a secondary amine represented by the general formula (5) and formaldehyde to form a 3-aminomethylindole derivative represented by the general formula (6); (c) further reacting the derivative with a peptide derivative represented by the formula (7).

More specifically, the present invention relates to a method of producing a compound, where comprising reacting a compound represented by the general formula (2):

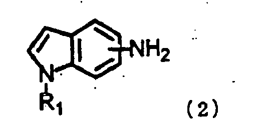

, wherein
R₁ is a C₁-C₆ straight-chain or branched alkyl group, a C₃-C₆ cycloalkyl group, a C₁-C₆ straight-chain or branched alkyl group substituted with a C₃-C₆ cycloalkyl group, or a C₇-C₁₂ aralkyl group which may be substituted with 1 to 3 substituents selected from a halogen atom, or a C₁-C₆ straight-chain or branched alkyl group;
-NH₂ group is attached to 4,5,6 or 7 position of the indole ring;
   with phenyl halocarbonate compound represented by the general formula (3):

R₄OCOX (3)

, wherein
R₄ is a phenyl group which may be substituted with selected from a C₁-C₆ straight-chain or branched alkyl group, a C₁-C₆ straight-chain or branched alkyloxy group or a halogen atom;
X is a halogen atom;
   to form a compound represented by the general formula (4):

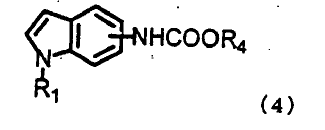

, wherein
R₁ and R₄ are the same as described above; and
-NHCOOR₄ group is attached to 4, 5, 6 or 7 position of the indole ring;
   followed by subjecting the compound to Mannich reaction with a secondary amine represented by the general formula (5):

(R₂)(R₃)NH (5)

, wherein
R₂ and R₃ are independently selected from a C₁-C₆ straight-chain or branched alkyl group; or
R₂ and R₃ are taken together with an adjacent nitrogen atom to form a 5 to 7 membered ring, 1 or 2 carbon atom(s) in the ring may be substituted with a nitrogen or an oxygen atom, and the ring may be substituted with a C₁-C₆ straight-chain or branched alkyl group;
   and formaldehyde to form a compound represented by the general formula (6):

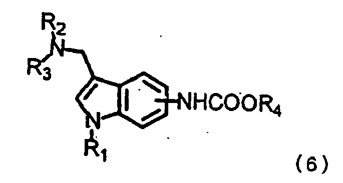

wherein,
R₁, R₂, R₃ and R₄ are the same as defined above, and
-NHCOOR₄ group is attached to the same position as above; and further reacting the compound with a peptide derivative represented by the general formula (7):

R5-NH-A₁-A₂-A₃-CO-N(R₆)(R₇) (7)

, wherein
each of R₆ and R₇ independently is a hydrogen atom; or a C₇-C₂₁ aralkyl group which may be substituted with 1 to 3 substituents selected from a halogen atom, or a C₁-C₆ straight-chain or branched alkyl group;
each A₁-A₂-A₃ independently represents a tripeptide residue consisting α-amino acid, selected from glycine, alanine, cyclohexylalanine, α,γ-diamino-butyric acid, lysine, arginine, phenylalanine, valine and naphtylalanine;
R₅ is a hydrogen atom, or a C₁-C₆ straight-chain or branched alkyl group;
   to produce a compound represented by the general formula (1):

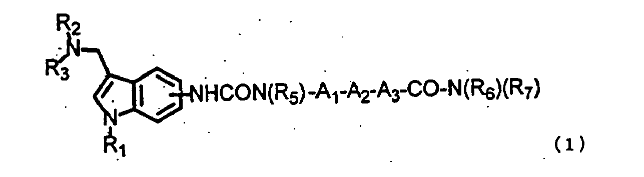

wherein,
R₁, R₂, R₃, R₅, R₆, R₇, A₁, A₂ and A₃ are the same as defined above; and
An ureylen group is attached to 4,5,6 or 7 position of the indole ring.
The present invention further relates to a method of producing an indole derivative, where comprising reacting a compound represented by the general formula (2):

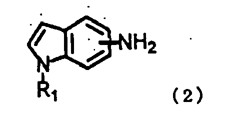

, wherein
R₁ is a C₁-C₆ straight-chain or branched alkyl group, a C₃-C₆ cycloalkyl group, a C₁-C₆ straight-chain or branched alkyl group substituted with a C₃-C₆ cycloalkyl group, or a C₇-C₁₂ aralkyl group which may be substituted with 1 to 3 substituents selected from a halogen atom, or a C₁-C₆ straight-chain or branched alkyl group;
-NH₂ group is attached to 4,5,6 or 7 position of the indole ring;
   with phenyl halocarbonate compound represented by the general formula (3):

R₄OCOX (3)

, wherein
R₄ is a phenyl group which may be substituted with selected from a C₁-C₆ straight-chain or branched alkyl group, a C₁-C₆ straight-chain or branched alkyloxy group or a halogen atom;
X is a halogen atom.
   to form a compound represented the general formula (4):

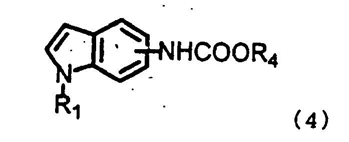

, wherein
R₁ and R₄ are the same as defined above; and
-NHCOOR₄ group is attached to 4,5, 6 or 7 position of the indole ring;
   followed by subjecting the compound to Mannich reaction with secondary amine represented by the general formula (5):

(R₂)(R₃)NH (5)

, wherein
R₂ and R₃ are independently selected from a C₁-C₆ straight-chain or branched alkyl group, or
R₂ and R₃ are taken together with an adjacent nitrogen atom to form a 5 to 7 membered ring, 1 or 2 carbon atom(s) in the ring may be substituted with a nitrogen or an oxygen atom, and the ring may be substituted with a C₁-C₆ straight-chain or branched alkyl group;
   and formaldehyde to form an indole derivative represented by the general formula (6):

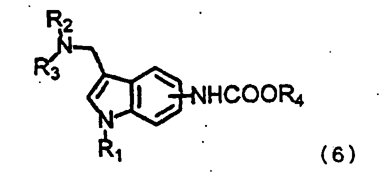

, wherein
R₁, R₂, R₃ and R₄ are the same as defined above, and
-NHCOOR₄ group is attached to the same position as above.
The present invention further relates to an indole derivative represented by the general formula (6):

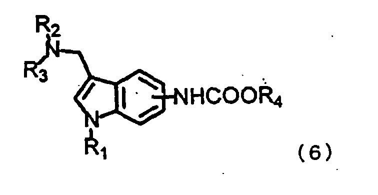

, wherein
R₁ is a C₁-C₆ straight-chain or branched alkyl group, a C₃-C₆ cycloalkyl group, a C₁-C₆ straight-chain or branched alkyl group substituted with a C₃-C₆ cycloalkyl group, or a C₇-C₁₂ aralkyl group which may be substituted with 1 to 3 substituents selected from a halogen atom, or a C₁-C₆ straight-chain or branched alkyl group;
R₂ and R₃ are independently selected from a C₁-C₆ straight-chain or branched alkyl group, or
R₂ and R₃ are taken together with an adjacent nitrogen atom to form a 5 to 7 membered ring, 1 or 2 carbon atom(s) in the ring may be substituted with a nitrogen or an oxygen atom, and the ring may be substituted with a C₁-C₆ straight-chain or branched alkyl group;
R₄ is a phenyl group which may be substituted with selected from a C₁-C₆ straight-chain or branched alkyl group, a C₁-C₆ straight-chain or branched alkyloxy group or a halogen atom; and
-NHCOOR₄ group is attached to 4, 5, 6 or 7 position of the indole ring.

The present invention also relates to the indole derivative, wherein R₁ in the general formula (6) is a C₁-C₆ straight-chain or branched alkyl group, a C₃-C₆ cycloalkyl group, a C₁-C₆ straight-chain or branched alkyl group substituted with a C₃-C₆ cycloalkyl group, or a benzyl group which may be substituted with 1 to 3 substituents independently selected from a halogen atom, or a C₁-C₆ straight-chain or branched alkyl group, and
R₄ is a phenyl group which may be substituted with a halogen atom.
The present invention further relates to the above described indole derivative, wherein -NHCOOR₄ group in the general formula (6) is attached to 5 or 6 position of the indole ring.

### Best Mode for Carrying out the Invention

The method for producing the (1,3-disubstituted-indolyl)urea derivative (1) and the intermediate thereof as well as the method for producing the same are shown in the following detailed description.
In the (1,3-disubstituted-indolyl)urea derivative or the intermediate thereof,
R₁ represents a C₁-C₆ straight-chain or branched alkyl group, a C₃-C₆ cycloalkyl group, a C₁-C₆ straight-chain or branched alkyl group substituted with a C₃-C₆ cycloalkyl group, or a C₇-C₁₂ aralkyl group substituted with 1 to 3 substituents selected from a halogen atom, or a C₁-C₆ straight-chain or branched alkyl group. The C₁-C₆ straight-chain or branched alkyl group is preferably a methyl group, an ethyl group, a n-propyl group, an isopropyl group, a n-butyl group, an isobutyl group, a sec-butyl group, a tert-butyl group, a n-pentyl group, a n-hexyl group and etc., more preferably is an isopropyl group. The C₃-C₆ cycloalkyl group is preferably a cyclohexyl group. The C₁-C₆ straight-chain or branched alkyl group substituted with a C₃-C₆ cycloalkyl group is preferably a cyclopropyl-methyl group and a cyclohexyl-methyl group, more preferably is a cyclohexyl-methyl group. The C₇-C₁₂ aralkyl group which may be substituted with 1 to 3 substituents selected from a halogen atom, or a C₁-C₆ straight-chain or branched alkyl group is preferably a benzyl group or a 2, 6-dichlorobenzyl group, more preferably is a 2,6-dichlorobenzyl group.

In the (1,3-disubstituted-indolyl) urea derivative (1) or the intermediate thereof,
R₂ and R₃ are independently selected from a C₁-C₆ straight-chain or branched alkyl group, or
R₂ and R₃ are taken together with an adjacent nitrogen atom to form a 5 to 7 membered ring, 1 or 2 carbon atom(s) in the ring may be substituted with a nitrogen or an oxygen atom, and the ring may be substituted with a C₁-C₆ straight-chain or branched alkyl group. The C₁-C₆ straight-chain or branched alkyl group is preferably a methyl group, an ethyl group, a n-propyl group, an isopropyl group, a n-butyl group, an isobutyl group, a sec-butyl group, a tert-butyl group, a n-pentyl group, a n-hexyl group, and etc., more preferably is a methyl group. The group, wherein R₂ and R₃ are taken together with an adjacent nitrogen atom to form a 5 to 7 membered ring, 1 or 2 carbon atom(s) in the ring may be substituted with a nitrogen or an oxygen atoms, and the ring may be substituted with a C₁-C₆ straight-chain or branched alkyl group, is preferably a piperidinyl group, a pyrrolidinyl group, a piperazinyl group, a morpholinyl group or a pyrrolidino group, more preferably is a pyrrolidinyl group, a pyrrolidino group or a piperazinyl group.

In the intermediate of the (1,3-disubstituted-indolyl)urea derivative (1), R₄ is a phenyl group which may be substituted with substituents selected from a C₁-C₆ straight-chain or branched alkyl group, a C₁-C₆ straight-chain or branched alkyloxy group or a halogen atom, particularly including such as a phenyl group, a 4-methylphenyl group, a 4-methoxyphenyl group and a 4-chlorophenyl group. R₄ is preferably a phenyl group or a 4-chlorophenyl group, and more preferably is a phenyl group.

In (1,3-disubstituted-indolyl)urea derivative(1) or the intermediate thereof, NO₂ group, NH₂ group, NHCOOR₄ group or the ureylen group is attached to 4,5,6 or 7 position of the indole ring, preferably 5 or 6 position, more preferably 5 position of the indole ring.
In the (1, 3-disubstituted-indolyl) urea derivative(1), each of R₆ and R₇ independently is a hydrogen atom; or a C₇-C₂₁ aralkyl group which may be substituted with 1 to 3 substituents selected from a halogen atom, or a C₁-C₆ straight-chain or branched alkyl group, particularly preferred is a hydrogen atom, a benzyl group or a benzhydryl group.
In the (1,3-disubstituted-indolyl)urea derivative(1), each A₁-A₂-A₃ independently represents tripeptide residue consisting of α-amino acids selected from natural and non-natural α-amino acids, preferably glycine, alanine, cyclohexylalanine, α,γ-diamino-butyric acid, lysine, arginine, phenylalanine, valine and naphtylalanine. The α-amino acid of A₁ is preferably glycine, alanine or cyclohexylalanine, more preferably is glycine. The α-amino acid of A₂ is preferably a C₃-C₈ chain diamino-carboxylic acid, more preferably are α,γ-diamino-butyric acid or lysine. The α-amino acid of A₃ is preferably phenylalanine, valine or β-naphtylalanine, more preferably is phenylalanine.

Each step in method for producing a (1,3-disubstituted indolyl)urea derivative (1) is shown as follows:

### 1. step 1

This is a step for producing the carbamate derivative represented by the general formula (4), by reacting 1-substituted-aminoindole derivative represented by the general formula (2) with phenyl halocarbonate compound represented by the general formula (3) in the presence of a base.
The base used herein include the organic bases such as triethylamine, N,N-diisopropylamine, pyridine, N,N-dimethylaniline, N,N-diethylaniline, 4-dimethylaminopiridine, piperidine, pyrrolidine, N-methylmorpholine, Hunig' s base, or the inorganic bases such as potassium carbonate, sodium carbonate, potassium hydrogen carbonate, sodium hydrogen carbonate, lithium hydride, potassium hydride, sodium hydride, preferably include organic bases, more preferably include N,N-diethylaniline or N,N-dimethylaniline.
The halogen atom of phenyl halocarbonate compound is chlorine, bromine or iodine, more preferably is chlorine or bromine.

This reaction can be conducted in the presence or absence of solvent, however, preferably is conducted in the solvent. The solvent can be any unless it takes part in the reaction including, for example, ethers such as diethyl ether, tetrahydrofuran, 1,4-dioxane, 1,3-dioxane, t-butylmethyl ether, monoglyme and diglyme, esters such as ethyl acetate and methyl acetate, aromatic hydrocarbons such as benzene, toluene, xylene, mesitylene, chlorobenzene and o-dichlorobenzene, aliphatic hydrocarbons such as n-hexane, cyclohexane, n-octane and n-decane, halogenated hydrocarbons such as dichloromethane, dichloroethane, chloroform and carbon tetrachloride, preferably are chloroform and dichloromethane, more preferably is dichloromethane. Those solvents can be used alone or in combination thereof. The volume of solvents is not particularly limited. The reaction temperature is from -20 to 50 °C, preferably from 0 to 25 °C, and the reaction period is from 0.5 to 16 hours, preferably from 1 to 5 hours.

### 2. step 2

This is a step for producing the 3-aminomethylindole derivative represented by the general formula (6), by reacting the carbamate derivative represented by the general formula (4) with the secondary amine compound represented by the general formula (5) and formaline in the presence of an acid.
The acid used herein includes an organic acids such as trifluoroacetic acid, propionic acid and acetic acids, or inorganic acids such as hydrochloric acid and sulfuric acid, preferably is an organic acid, more preferably is propionic acid or acetic acid.
The reaction can be conducted in the presence or absence of solvent, however, preferably is conducted in the solvent. The solvents used herein include ethers such as diethyl ether, tetrahydrofuran, 1,4-dioxane, 1,3-dioxane, t-butylmethyl ether, monoglymeanddiglyme, alcohols such as methanol and ethanol, esters such as ethyl acetate and methyl acetate, aromatic hydrocarbons such as benzene, toluene, xylene, mesytylene, chlorobenzene and o-dichlorobenzene, aliphatic hydrocarbons such as n-hexane, cyclohexane, n-octane and n-decane, halogenated hydrocarbons such as dichloromethane, dichloroethane, chloroform and carbon tetrachloride, wherein preferred are ethers such as diethyl ether, tetrahydrofuran, 1,4-dioxane, 1,3-dioxane, t-butylmethyl ether, monoglyme and diglyme, and more preferred is 1, 4-dioxane. Those solvents can be used alone or in combination thereof. The volume of solvents is not particularly limited. The reaction temperature is from 0 to 50 °C, preferably from 10 to 25 °C, and the reaction period is from 0.5 to 24 hours, preferably from 3 to 18 hours.

The compound represented by the general formula (6) exhibits good crystalline features, and can be easily purified by recrystalization. The compound of interest can be obtained in high yield and high purity according to the above steps when conducted at industrial scale, efficiently reflecting the small scale reaction.
For example, the compound represented by the following formula:

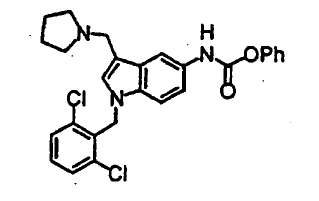

, wherein R₁ is 2, 6-dichlorobenzyl group, R₂ and R₃ are taken together with an adjacent nitrogen atom to form a tetramethylene group, R₄ is a phenyl group, and phenoxycarbonylamino group is attached to the 5 position of the indole ring;

exhibits crystalline features, can be stored for over 2 years at room temperature under light shielding, has high reactivity in urea reaction, and is an extremely useful intermediate for industrial production.
The compound represented by the following formula:

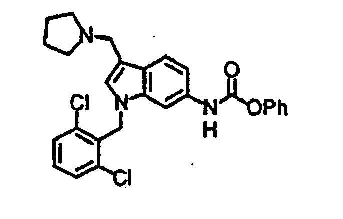

wherein R₁ is 2, 6-dichlorobenzyl group, R₂ and R₃ are taken together with an adjacent nitrogen atom to form a tetramethylene group, R₄ is a phenyl group, and phenoxycarbonylamino group is attached to the 6 position of the indole ring,

is amorphous powder, however, exhibits stability over 3 months of storage under refrigeration and light shielding, has high reactivity inurea reaction, and is an extremelyuseful intermediate for industrial production.

### 3. Step 3

This is a step for producing a compound of interest represented by the general formula (1), by reacting a 3-aminomethylindole derivative represented by the general formula (6) with a peptide derivative represented by the general formula (7) in the presence of a base.
The base used herein include organic bases such as triethylamine, N,N-diisopropylamine, pyridine, N,N-dimethyl-aniline, 4-dimethylaminopiridine, piperidine, pyrrolidine, N-methylmorpholine and Hunig's base, or inorganic bases such as potassium carbonate, sodium carbonate, potassium hydrogen carbonate, sodium hydrogen carbonate, lithium hydride, potassium hydride and sodium hydroxide, wherein preferred are organic bases, and more preferred are Hunig's base and triethylamine.

The reaction can be conducted in the presence or absence of solvents, however, preferably is conducted in the solvent. The solvents can be any unless it takes part in the reaction including, for example, ethers such as diethyl ether, tetrahydrofuran, 1,4-dioxane, 1,3-dioxane, t-butylmethyl ether, monoglyme and diglyme, esters such as ethyl acetate and methyl acetate, aromatic hydrocarbons such as benzene, toluene, xylene, mesitylene, chlorobenzene and o-dichlorobenzene, aliphatic hydrocarbons such as n-hexane, cyclohexane, n-octane and n-decane, halogenated hydrocarbons such as dichloromethane, dichloroethane, chloroform, and carbon tetrachloride, wherein preferred are chloroform and 1,2-dichloroethane, and more preferred is 1,2-dichloroethane. The solvents can be used alone or in combination thereof. The volume of solvents is not particularly limited. The reaction temperature is from 25 to 120 °C, preferably from 70 to 100 °C, and the reaction period is from 1 to 24 hours, preferably from 3 to 16 hours.

The present invention is described in more detail by the following examples, however, the scope of the technical field to which it relates should not be construed as limited thereto.

### EXAMPLE 1

Preparation of 5-phenyloxycarbonylamino-N-[1-(2,6 -dichlorophenyl)methyl]-1H-indole

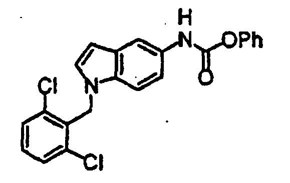

Phenyl chloroformate (1.18 g, 7.56 mmol) in anhydrous dichloromethane (5 mL) was added dropwise to a solution of 5-amino-N-[1-(2,6-dichlorophenyl)methyl]-1H-indole (2.0 g, 6.87 mmol) and N,N-dimethylaniline (916 mg, 7.56 mmol) in anhydrous dichloromethane (25 mL) under ice-cooling. The mixture was stirred at room temperature for 2 hours, diluted with water, and extracted with chloroform. The organic layer was washed sequentially with 1N hydrochloric acid, saturated aqueous sodium bicarbonate and brine, and dried over anhydrous sodium sulfate. The solvent was distilled off to give a crude crystalline product. The crude crystal was then purified by recrystallization from chloroform-hexane to give the title compound as light off-white needles (2.55 g, yield 90 %) .
¹H-NMR (CDCl₃) δ:
5.52 (s, 2H), 6. 43 (d, J=3.2Hz, 1H), 6. 92 (bs, 1H), 6. 95 (d, J=3.2Hz, 1H), 7.19-7.31 (m, 5H), 7.35-7.42 (m, 4H), 7.47 (d, J=8.9Hz, 1H), 7.72 (bs, 1H)

### EXAMPLE 2

Preparation of 5-phenyloxycarbonylamino-3-(1-pyrrolidinylmethyl)-N-[1-(2,6-dichlorophenyl)methyl]-1H-indole

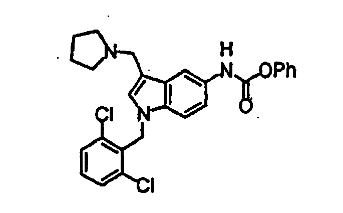

5-phenyloxycarbonylamino-N-[1-(2,6-dichlorophenyl)methyl ]-1H-indole (1.20 g, 2.92 mmol) in methanol (15 mL) was added to a solution of pyrrolidine (2.07 g, 29.2 mmol), acetic acid (2.80 g, 46.7 mmol) and 37 % aqueous formaldehyde solution (1.73 g, 21.3 mmol) in 1,4-dioxane (5 mL) at room temperature. The mixture was stirred at room temperature for 6 hours, diluted with water, and extracted with chloroform. The organic layer was washed with brine, and dried over anhydrous sodium sulfate. The solvent was distilled off to give a crude crystalline product. The crude crystal was purified by silica gel chromatography (chloroform/methanol 30:1) to give pale yellow powder (1.38 g, yield 95 %). The resultant was then purified by recrystallization from chloroform-hexane to give the title compound as pale yellow powder (940 mg, yield 65 %).
Melting point: 189-196 °C (decomposition)
¹H-NMR (CDCl₃) δ:
1.93 (br, 2H), 2.13 (br, 2H), 2.84 (br, 2H), 3.72 (br, 2H), 4.29 (d, J=4.8Hz, 2H), 5.55 (s, 2H), 7.15-7.43 (m, 9H), 7.44 (d, J=7. 7Hz, 1H), 7.55 (s, 1H), 7.99 (bs, 1H), 12.29 (br, 1H) IR(KBr)cm-1 :1693, 1563, 1191, 1179, 1154, 1105
Mass(FAB) : 494 496

### EXAMPLE 3

Preparation of 6-phenyloxycarbonylamino-N-[1-(2,6-dichlorophenyl)methyl]-1H-indole

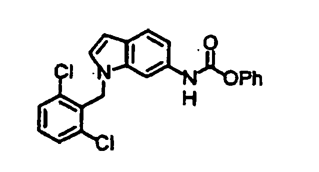

Phenyl chloroformate (173 mg, 1.1 mmol) in anhydrous dichloromethane (2 mL) was added dropwise to a solution of 6-amino-N-[1-(2,6-dichlorophenyl)methyl]-1H-indole (291 mg, 1.0 mmol) and N,N-dimethylaniline (133 mg, 1.1 mmol) in anhydrous dichloromethane (5 mL) under ice-cooling. The mixture was stirred at room temperature for 2 hours, diluted with water, and extracted with chloroform. The organic layer was washed sequentially with 1N hydrochloric acid, saturated aqueous sodium bicarbonate and brine, and dried over anhydrous sodium sulfate. The solvent was distilled off to give a crude crystalline product. The crude crystal was purified by recrystallization from chloroform-hexane to give the title compound as light off-white needles (385 mg, yield 93 %) .
¹H-NMR (CDCl₃) δ:
5.47 (s, 2H), 6.42 (d, J=2.6Hz, 1H), 6.84 (bs, 1H), 6.93 (dd, J=1.7, 8.9Hz, 1H), 7.04 (bs, 1H), 7.20-7.30 (m, 4H), 7.36-7.44 (m, 4H), 7.53 (d, J=8.9Hz, 1H), 8.02 (bs, 1H)

### EXAMPLE 4

Preparation of 6-phenyloxycarbonylamino-3-(1-pyrrolidinylmethyl)-N-[1-(2,6-dichlorophenyl)methyl]-1H-indole

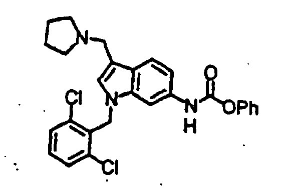

6-phenyloxycarbonylamino-N-[1-(2,6-dichlorophenyl)methyl ]-1H-indo (247 mg, 0.6 mmol) in methanol (3 mL) was added to a solution of pyrrolidine (427 mg, 0.6 mmol), acetic acid (576 mg, 9.6 mmol) and 37 % aqueous formaldehyde solution (355 mg, 4.38 mmol) in 1,4-dioxane (1 mL) at room temperature. The mixture was stirred at room temperature for 16 hours, diluted with water, and extracted with chloroform. The organic layer was washed with brine, and dried over anhydrous sodium sulfate. The solvent was distilled off to give a crude crystalline product. The crude crystal was purified by silica gel chromatography (chloroform/methanol 20:1, followed by chloroform/methanol (ammonia) 20: 1) to give the title compound as slightly pink amorphous powder(94 mg, yield 99 %).
¹H-NMR (CDCl₃) δ :
1.98 (br, 4H), 2.80 (br, 4H), 4.24 (s, 2H), 5.49 (s, 2H), 7.08 (dd, J=1.7,8.5Hz, 1H), 7.13 (bs, 1H), 7.21-7.32 (m, 5H), 7.38 -7.44 (m, 4H), 7.58 (d, J=8.5Hz, 1H), 8.02 (bs, 1H) IR(KBr)cm-1:1732, 1488, 1437, 1334, 1196
Mass(FAB): 494 496

### EXAMPLE 5

Preparation of [[N-[1-(2,6- dichlorophenyl)methyl]-3-(1-pyrrolidinylmethyl)-1H-indole-5-yl]aminocarbonyl]-glycine-N-ω -t-butoxycarbony-L-lysine-L-phenylalanine-N-benzhydrylamido

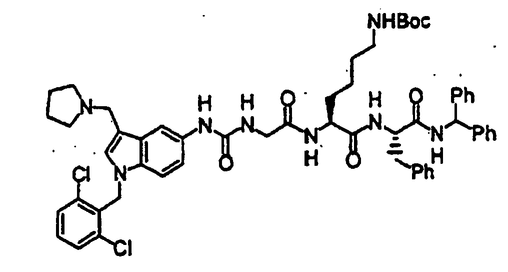

Glycine-N-ω-t-butoxycarbony-L-lysine-L-phenylalanine-N-b enzhydrylamido (203 mg, 0.33 mmol) and triethylamine (91 mg, 0.9 mmol) were added to a solution of 5-phenyloxycarbonylamino-3-(1-pyrrolidinylmethyl)-N-[1-(2,6-d ichlorophenyl)methyl]-1H-indole (150 mg, 0.3 mmol) in dichloroethane (20mL). The mixture was stirred at 80 °C for 3 hours, concentrated *in vacuo,* and the residue was recrystallized from chloroform-methanol-ether to give the title compound(287mg,yield 94 %) as light yellow solid.
¹H-NMR (DMSO-d₆,120°C) δ:
1.15-1.30 (m, 2H), 1.30-1.40(m, 2H), 1.37 (s, 9H), 1.40-1.55 (m, 1H), 1.55-1.65 (m, 1H), 1.87 (br, 4H), 2.50-2.65 (m, 2H), 2.89 (overlapped with H₂O, 2H), 3.04 (dd, J= 5.8, 13.9Hz, 1H), 3.10 (br, 4H), 3.76 (d, J=5.3Hz, 2H), 4.22 (dt, J=5. 3, 8.0Hz, 1H), 4.26 (s, 2H), 4.65 (dt, J=5.3, 8.0Hz, 1H), 5.35 (s, 2H), 6.07 (d, J=8.2Hz, 2H), 6.26 (br, 1H), 7.10-7.30 (m, 15H), 7.40-7.46 (m, 2H), 7.53 (d, J=7.5Hz, 2H), 7.61 (d, J=7.5Hz, 1H), 7.69 (d, J=8.0Hz, 1H), 7.77 (d, J=1.9Hz, 1H), 8.32 (d, J=8.0Hz, 1H)

### EXAMPLE 6

Preparation of [[N-[1-(2,6- dichlorophenyl)methyl]-3-(1-pyrrolidinylmethyl)-1H-indole-5-yl]aminocarbonyl]-glycine-L-lysine-L-phenylalanine-N-benzhydrylamido

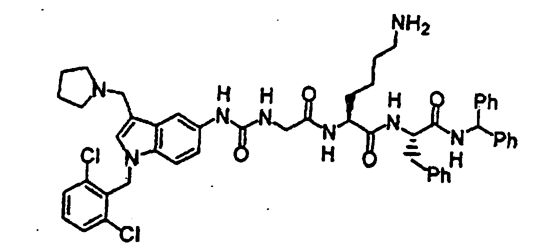

Trifluoroacetic acid (ImL, 13.15 mmol) was added slowly to a solution of [[N-[1-(2,6-dichlorophenyl)methyl]-3-(1-pyrrolidinylmethyl)-1H-indole-5-y 1]aminocarbonyl]-glycine-N-ω-t-butoxycarbony-L-lysine-L-pheny lalanine-N-benzhydrylamido (34 mg, 0. 033 mmol) in dichloromethane (2 mL) under ice-cooling, and stirred for 0.5 hour. The reaction solution was neutralized and made basic (pH= 12) with 2N sodium hydroxide under ice-cooling, and extracted with chloroform. The organic layer was washed with saturated aqueous sodium bicarbonate and brine, and dried over anhydrous sodium sulfate. The solvent was distilled off to give a crude crystalline product. The crude crystal was purified by silica gel chromatography, and recrystallized from the chloroform-methanol-ether to give the title compound (23 mg, yield 75 %) as light yellow solid.
Melting point: 207-215 °C (decomposition)
¹H-NMR (DMSO-d₆, 120°C) δ:
1.17-1.37 (m, 4H), 1.43-1.54 (m, 1H), 1.54-1.68 (m, 1H), 1.63 (br, 4H), 2.43 (br, 4H), 2.46-2.53 (m, 2H), 2.80-2.92 (m, 1H), 3.05 (dd, J=5.6, 13.9 Hz, 1H), 3.61 (s, 2H), 3.73 (d, J=4.8Hz, 2H), 4.22 (br, 1H), 4.65 (br, 1H), 5.46 (s, 2H), 6.10 (br, 1H) 6.80 (s, 1H), 7.08-7.30 (m, 19H), 7.30 (d, J=8.7Hz, 1H), 7.40 (dd, J=7.3, 8.8Hz, 1H), 7.51 (d, J=7.5Hz, 2H), 7.59 (d, J=1.9Hz, 1H), 8.20 (br, 2H)
IR(KBr)cm-1 :3281, 1639, 1542, 1493, 1437, 699
Mass(FAB) : 915 917

### EXAMPLE 7

Preparation of [[N-[1-(2,6- dichlorophenyl)methyl]-3-[1-(4-methylpiperazinyl)methyl]-1H-indole-5-yl]aminocarbonyl] -glycine-L-lysine-L-phenylalanine-N-benzhydrylamido

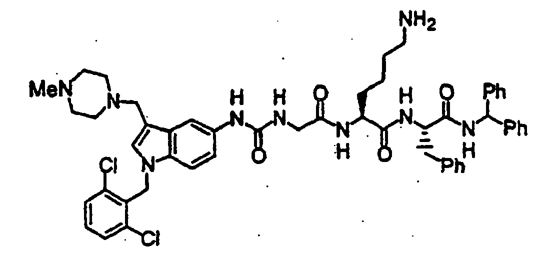

5-phenyloxycarbonylamino-N-[1-(2,6-dichlorophenyl)methyl ]-1H-indole (15 mg, 0.036 mmol) in methanol (1.0 mL) was added to a solution of 1-methylpiperazine (36 mg, 0.36 mmol), acetic acid (35 mg, 0.58 mmol) and 37 % aqueous formaldehyde solution (22 mg, 0.27 mmol) in 1,4-dioxane (0.5 mL) at room temperature as same as the example 2. The mixture was stirred at room temperature for 16 hours, and further stirred at 50 °C for 2 hours. Then the reaction solution was diluted with water, and extracted with chloroform. The organic layer was washed with brine, and dried over anhydrous sodium sulfate. The solvent was distilled off to give crude oil. The oil was purified by silica gel chromatography (chloroform/methanol(ammonia) 40:1) to give 5-phenyloxycarbonylamino-3-(4-methylpiperazyinyl)methyl-N-[1-(2,6-dichlorophenyl)methyl] -1H-indol (19 mg, yield 100 %) as colorless amorphous powder.
¹H-NMR (CDCl₃) δ:
2.24 (s, 3H), 2.30-2.65(br, 8H), 3.61 (s, 2H), 5.47 (s, 2H), 6.88 (s, 1H), 7.05-7.35 (m, 6H), 7.35-7.45 (m, 5H), 7.75 (bs, 1H)
The title compound was continuously synthesized with the above product according to the same method of example 5-6.
¹H-NMR (CDCl₃+CD₃OD) δ:
1.12-1.29 (m, 2H), 1.30-1.42 (m, 2H), 1.46-1.58 (m, 1H), 1.60 -1.72 (m, 1H), 2.23 (s, 3H), 2.41 (bs, 4H), 2.49 (bs, 4H), 2.53 -2.58 (m, 2H), 2.91-2.97 (m, 1H), 3.09-3.21 (m, 1H), 3.58 (s, 2H), 3.75 (d, J=2.1Hz, 1H), 4.20-4.31 (m, 1H), 4.64-4.73 (m, 1H), 5.47 (s, 2H), 6.13 (s, 1H), 6.88 (s, 1H), 7.02-7.30 (m, 16H), 7.30-7.40 (m, 4H), 7.53 (d, J=1.7Hz, 1H)

### EXAMPLE 8

Preparation of [[N-[1-(2,6-dichlorophenyl) methyl]-1H-indole-5-yl]aminocarbonyl]-N-methylglycine-L-α, γ-diaminobutyric acid-L-phenylalanine-N-benzylamido

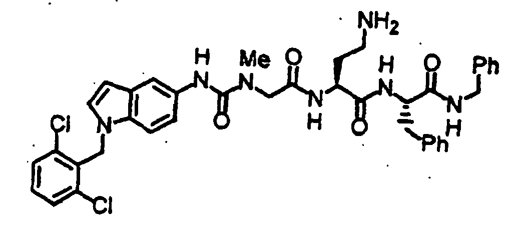

By the same manner as Examples 1, 2, 5 and 6, the title compound was synthesized by using 5-phenyloxycarbonylamino-N-[1-(2,6-dichlorophenyl)methyl]-1H-indole instead of 5-phenyloxycarbonylamino-3-(1-pyrrolidinylmethyl)- N-[1-(2,6-dichlorophenyl)methyl] -1H-indol on the indole ring portion, and methylglycine-N -ω-t-butoxycarbonyl-L-α,γ-diaminobutyric acid-L-phenylalanine-N-benzylamido instead of glycine-N-ω-t-butoxycarbonyl-L-lysine-L-pheiylalanine-N-benzh ydrylamido.
¹H-NMR (CDCl₃) δ :
1.69-1.78 (m, 2H), 2.04-2.65 (m, 2H), 3.12 (s, 3H), 3.24-3.36 (m, 2H), 3.72 (s, 2H), 4.24-4.26 (m, 3H), 4.50-4.61 (m, 1H), 4.75 -4.88(m,1H),5.47(s,2H),6.42 (d, J=2. 9Hz, 1H), 6. 92 (d, J=2. 9Hz, 1H), 6.84-7.62 (m, 13H), 9.60 (bs, 1H)

### EXAMPLE 9

Preparation of [[N-1-isopropyl-3-(1-pyrrolidinylmethyl) -1H-indole-5-yl]aminocarbonyl]-glycine-L-α, γ-diaminobutyric acid-L-phenylalanine-N-benzylamido

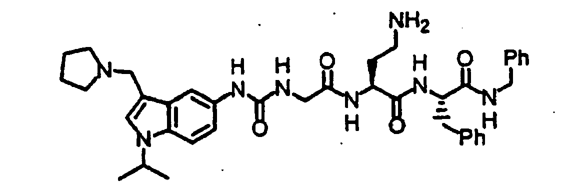

By the same manner as Examples 1,2,5 and 6, the title compound was synthesized by using 5-phenyloxycarbonylamino-N-1-isopropyl-3-(1-pyrrolidinylmethy 1)-1H-indole in stead of 5-phenyloxycarbonylamino-3-(1-pyrrolidinylmethyl)-N-[1-(2,6-dichlorophenyl)methyl]-1H-indole on the indole ring portion, and glycine-N-γ-t-butoxycarbonyl-L-α,γ-diaminobutyric acid-L-phenylalanine-N-benzylamido in stead of glycine-N-ω-t-butoxycarbonyl-L-lysine-L-phenylalanine-N-benzhydrylamido.
¹H-NMR (DMSO-d₆,120°C) δ:
1.42 (d, J=6.5Hz, 6H), 1.56-1.78 (m, 2H), 1.68 (bs, 4H), 2.49 (bs, 4H), 2.54-2.60 (m, 2H), 2.85-2.96 (m, 1H), 3.07 (dd, J=5.8, 14.1Hz, 1H), 3.68 (s, 2H), 3.74 (d, J=4.6Hz, 2H), 4.23-4.30 (m, 2H), 4.30-4.36 (m, 1H), 4.54 (dd, J=5.8, 8.7Hz, 1H), 4.59 (qq, J=6.5, 6.5Hz, 1H), 7.10-7.30 (m, 13H), 7.58 (bs, 1H)

### EXAMPLE 10

Preparation of [[N-[1-(2,6-dichlorophenyl)methyl]-3-(1-pyrrolidinylmethyl)-1 H-indole-6-yl]aminocarbonyl]-glycine-N-γ-t-butoxycarbonyl-L-α ,γ-diaminobutyric acid-L-phenylalanine-N-benzylamido

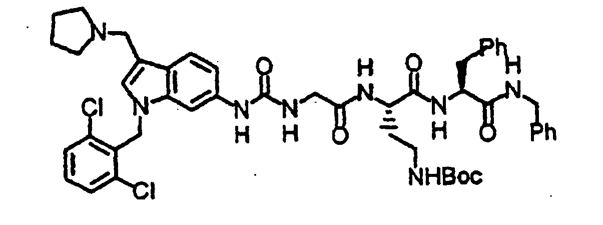

Glycine-N-γ-t-butoxycarbony-L-α,γ-diaminobutyric acid-L-phenylalanine-N-benzylamido (28 mg, 0.055 mmol) and triethylamine (25 mg, 0.25 mmol) were added to a solution of 6-phenyloxycarbonylamino-3-(1-pyrrolidinylmethyl)-N-[1-(2,6-d ichlorophenyl)methyl]-1H-indole (25 mg, 0.05 mmol) in dichloroethane (4 mL). The mixture was stirred at 80 °C for 3 hours, and concentrated *in vacuo.* The residue was recrystallized from the chloroform-methanol-ether to give the title compound (42 mg, yield 92 %) as light off-white solid.
¹H-NMR(CD₃OD) δ:
1.41 (s, 9H), 1.42(bs, 4H), 1.56-1.68 (m, 1H), 1.75-1.90 (m, 1H), 1.96 (bs, 4H), 2.90-3.05 (m, 1H), 3.10-3.25 (m, 1H), 3.35-3.60 (m, 2H), 3.79 (d, J= 16.3Hz, 1H), 3.92 (d, J=16.3Hz, 1H), 4.19 -4.37 (m, 3H), 4.40 (s, 2H), 4.50-4.59 (m, 1H), 5.42 (ABq, J=16. 7Hz, 2H), 7.03 (s, 1H), 7.10-7.30 (m, 13H), 7.38 (dd, J=1.7, 8.5Hz, 1H), 7.48 (d, J=0.97 1H), 7.60 (d, J=8.5Hz, 1H), 7.87 (d, J=1.7Hz, 1H)

### EXAMPLE 11

Preparation of [[N-[1-(2,6- dichlorophenyl)methyl]-3-(1-pyrrolidinylmethyl)-1H-indole-6-yl]aminocarbonyl]-glycine-L-α,γ-diaminobutyric acid-L-phenylalanine-N-benzylamido

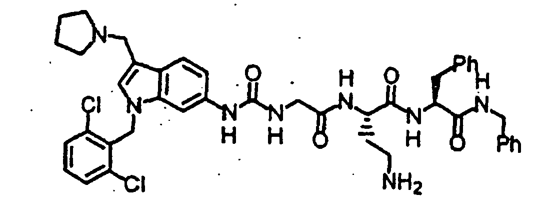

Trifluoroacetic acid (0.5 mL, 6.57 mmol) was added slowly to a solution of [[N-[1-(2,6- dichlorophenyl)methyl]-3-(1-pyrrolidinylmethyl)-1H-indole-6-yl]aminocarbonyl]-glycine-N-γ-t-butoxycarbony-L-α,γ-diaminobutyric acid-L-phenylalanine -N-benzylamido (37 mg, 0.04 mmol) in dichloromethane (1.5 mL) under ice-coooling, and stirred for 0.5 hour. The reaction solution was neutralized and made basic (pH=12) with 2N sodium hydroxide under ice-cooling, and extracted with chloroform. The organic layer was washed with saturated aqueous sodium bicarbonate and brine, and dried over anhydrous sodium sulfate. The solvent was distilled off to give a crude crystalline product. The crude crystal was purified by silica gel chromatography, and recrystallized from the chloroform-methanol-ether to give the title compound (19 mg, yield 59 %) as white solid.
¹H-NMR (CDCl₃-CD₃OD) δ:
1.62-1.74 (m, 2H), 1.74 (bs, 4H), 2. 00-2. 24 (m, 1H), 2.46-2.55 (m, 1H), 2.57 (bs, 4H), 2.93 (dd, J=9.7, 13.3Hz, 1H), 3.28 (dd, J=5.3, 13.3Hz, 1H) 3.65 (s, 2H), 4.27-4.46 (m, 5H), 4.68 (dd, J=5.3, 9.7Hz, 1H), 5.39 (ABq, J=16.3Hz, 2H), 6.78 (s, 1H), 6.96 (dd, J=1.7, 8.2Hz, 1H), 7.16-7.41(m, 14H), 7.55 (d, J=8.2Hz, 1H), 7.77 (d, J=0.6Hz, 1H)
   IR(KBr)cm-1:3282, 1632, 1542, 1454, 1233, 697
   Mass(FAB): 811 813

### EXAMPLE 12

Preparation of [N-(1-isopropyl-1H-indole-5-yl) aminocarbonyl]-glycine-L-α, γ-diaminobutyric acid-L-phenylalanine-N-benzylamido

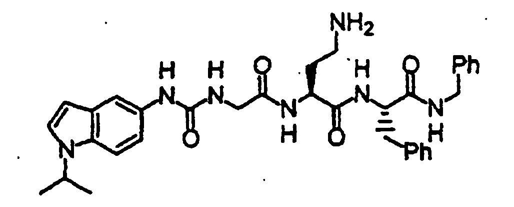

By the same manner as Examples 1, 2, 5 and 6, the title compound was synthesized by using 5-phenyloxycarbonylamino-N-isopropyl-1H-indole instead of 5-phenyloxycarbonylamino-3-(1-pyrrolidinylmethyl)-N-[1-(2,6-dichlorophenyl)methyl]-1H-indole on the indole ring portion, and glycine-N-ω-t-butoxycarbonyl-L-α,γ-diaminobutyric acid-L-phenylalanine-N-benzylamido instead of glycine-N-ω-t-butoxycarbonyl-L-lysine-L-phenylalanine-N-benzh ydrylamido.
¹H-NMR (DMSO-d₆, 120°C) δ:
1.44 (d, J=6.3Hz, 6H), 1.50-1.63 (m, 1H), 1.63-1.75 (m, 1H), 2,45 -2.60 (m, 2H), 2,90-3,25 (m, 2H), 3.72 (bs, 2H), 4.25 (bs, 2H), 4.25-4.45 (m, 1H), 4.50-4.60 (m, 1H), 4.60-4.75 (m, 1H), 6.13 (bs, 1H), 6.30 (bs, 1H), 7.02-7.35 (m, 13H), 7.55 (bs,1H), 7.85 (br, 1H)

### INDUSTRIAL APPLICABILITY

The present invention can provide a useful industrial method for producing a (1,3-disubstituted indolyl)urea derivative, and can provide an intermediate thereof as well as the method for producing the same.
Particularly the compound represented by the general formula (6) of the present invention has a sufficient reactivity in urea synthesis reaction, beside that it is quite stable. The present invention provides a compound which is an extremely important industrial intermediate for urea synthesis. The present invention is to provide an effective method for producing N-indolyl urea derivatives, and thus industrially applicable.

## Claims

1. A method for producing a compound represented by the general formula (1) : 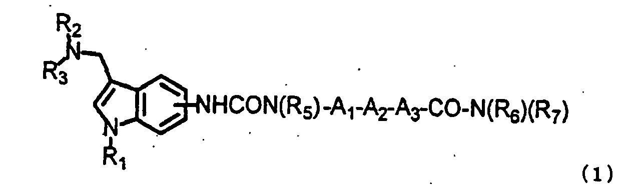 [wherein:
R₁ is a C₁-C₆ straight-chain or branched alkyl, a C₃-C₆ cycloalkyl group, a C₁-C₆ straight-chain or branched alkyl group substituted with a C₃-C₆ cycloalkyl group, or a C₇-C₁₂ aralkyl group which may be substituted with 1 - 3 substituents selected from a halogen atom or a C₁-C₆ straight-chain or branched alkyl group;
R₂ and R₃ are independently selected from a C₁-C₆ straight-chain or branched alkyl group, or
R₂ and R₃ are taken together with the adjacent nitrogen atom to form a 5 to 7 membered ring, 1 or 2 carbon atom(s) in the ring may be substituted with a nitrogen or an oxygen atom, or the said ring may be substituted with a C₁-C₆ straight-chain or branched alkyl group;
R₅ is a hydrogen atom, or a C₁-C₆ straight-chain or branched alkyl group;
R₆ and R₇ are independently a hydrogen atom or a C₇-C₂₁ aralkyl group which may be substituted with 1 - 3 substituents selected from a halogen atom or a C₁-C₆ straight-chain or branched alkyl group;
A₁-A₂-A₃ is a tri-peptide residue consisting of A₁, A₂ and A₃, each of which is α-amino acid independently selected from glycine, alanine, cyclohexylalanine, α,γ-diamino-butyric acid, lysine, arginine, phenylalanine, valine or naphtylalanine; and
An ureylen group is attached to 4, 5, 6 or 7 position of the indole ring], wherein
the method comprises the following steps:
(a) reacting a compound represented by the general formula (2): 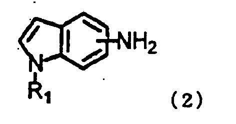 [wherein:
R₁ is the same as defined above,
-NH₂ group is attached to 4, 5, 6 or 7 position of the indole ring], with phenyl halocarbonate compound represented by the general formula (3):
R₄OCOX (3)
[wherein:
R₄ is a phenyl group which may be substituted with a C₁-C₆ straight-chain or branched alkyl group, a C₁-C₆ straight-chain or branched alkyloxy group or a halogen atom;
X is a halogen atom],
to form a compound represented the general formula (4): 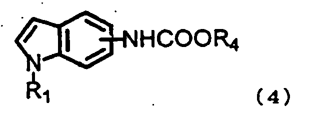 [wherein:
R₁ and R₄ are the same as defined above; and
-NHCOOR₄ group is attached to 4,5,6 or 7 position of the indole ring]; followed by
(b) subj ecting to Mannich reaction with a secondary amine compound represented by the general formula (5):
(R₂)(R₃)NH (5)
[wherein R₂ and R₃ are the same as defined above], and formaldehyde to form a compound represented by the general formula (6): 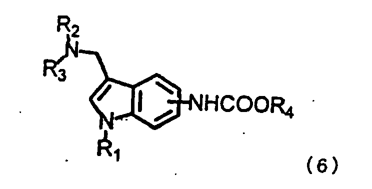 [wherein:
R₁, R₂, R₃ and R₄ are the same as defined above, and
-NHCOOR₄ group is attached to the same position of the indole ring as indicated above], followed by
(c) reacting with a peptide derivative represented by the general formula (7):
R₅-NH-A₁-A₂-A₃-CO-N(R₆)(R₇) (7)
[wherein R₆,R₇,A₁,A₂,A₃ and R₅ are the same as defined above] .

2. A method for producing an indole derivative represented by the general formula (6): 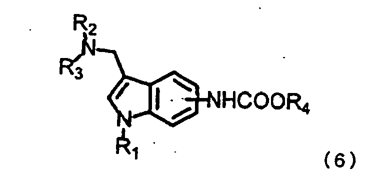 [wherein:
R₁ is a C₁-C₆ straight-chain or branched alkyl group, a C₃-C₆ cycloalkyl group, a C₁-C₆ straight-chain or branched alkyl group substituted with a C₃-C₆ cycloalkyl group, or a C₇-C₁₂ aralkyl group which may be substituted with 1 - 3 substituents selected from a halogen atom or a C₁-C₆ straight-chain or branched alkyl group;
R₂ and R₃ are independently selected from a C₁-C₆ straight-chain or branched alkyl group or;
R₂ and R₃ are taken together with the adjacent nitrogen atom to form a 5 to 7 membered ring, wherein 1 or 2 carbon atom(s) in the ring may be substituted with a nitrogen or an oxygen atom, or the said ring may be substituted with a C₁-C₆ straight-chain or branched alkyl group;
R₄ is a phenyl group which may be substituted with a C₁-C₆ straight-chain or branched alkyl group, a C₁-C₆ straight-chain or branched alkyloxy group or halogen atoms; and
-NHCOOR₄ group is attached to 4,5,6 or 7 position of the indole ring], wherein the method comprises the following steps:
(a) reacting a compound represented by the general formula (2): 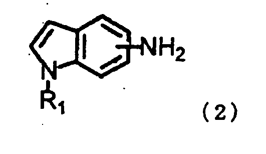 [wherein:
R₁ is the same as defined above;
-NH₂ group is attached to 4, 5, 6 or 7 position of the indole ring], with phenyl halocarbonate compound represented by the general formula (3):
R₄OCOX (3)
[wherein:
R₄ is the same as defined above;
X is a halogen atom],
to form a compound represented the general formula (4): 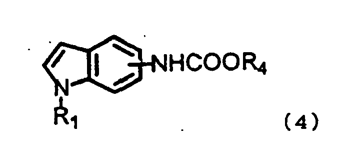 [wherein:
R₁ and R₄ are the same as defined above; and
-NHCOOR₄ group is attached to the same position of the indole ring as indicated above], followed by
(b) subj ecting to Mannich reaction with a secondary amine compound represented by the general formula (5):
(R₂)(R₃)NH (5)
[wherein R₂ and R₃ are the same as defined above]; and formaldehyde to form a compound represented by the general formula (6).

3. An indole derivative represented by the general formula (6): 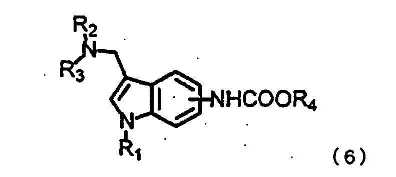 [wherein:
R₁ is a C₁-C₆ straight-chain or branched alkyl group, a C₃-C₆ cycloalkyl group, a C₁-C₆ straight-chain or branched alkyl group substituted with a C₃-C₆ cycloalkyl group, or a C₇-C₁₂ aralkyl group which may be substituted with 1 - 3 substituents selected from a halogen atom or a C₁-C₆ straight-chain or branched alkyl group;
R₂ and R₃ are independently selected from a C₁-C₆ straight-chain or branched alkyl group, or
R₂ and R₃ are taken together with the adjacent nitrogen atom to form a 5 to 7 membered ring, 1 or 2 carbon atom(s) in the ring may be substituted with a nitrogen or an oxygen atom, or the said ring is may be substituted with a C₁-C₆ straight-chain or branched alkyl group;
R₄ is a phenyl group which may be substituted with a C₁-C₆ straight-chain or branched alkyl group, a C₁-C₆ straight-chain or branched alkyloxy group or a halogen atom; and
-NHCOOR₄ group is attached to 4, 5, 6 or 7 position of the indole ring].

4. The indole derivative of formula (6) according to claim 3, wherein
R₁ is a C₁-C₆ straight-chain or branched alkyl group; a C₃-C₆ cycloalkyl group; a C₁-C₆ straight-chain or branched alkyl group substituted with a C₃-C₆ cycloalkyl; or a benzyl group which may be substituted with 1 - 3 substituents selected from a halogen atom, or a C₁-C₆ straight-chain or branched alkyl group; and
R₄ is a phenyl group may be substituted with a halogen atom. 5. The indole derivative of formula (6) according to claim 3 or 4, wherein -NHCOOR₄ group is attached to 5 or 6 position of the indole ring.
